# EUROPÄISCHE PATENTSCHRIFT

(11) **EP 3 178 419 B1**
(45) Veröffentlichungstag und Bekanntmachung des Hinweises auf die Patenterteilung: **12.06.2019**
(21) Anmeldenummer: 16200000.4
(22) Anmeldetag: 22.11.2016
(51) Int. Cl.: A61B 17/29, A61B 17/00, A61B 90/00

(54) **KOPPLUNGSEINRICHTUNG FÜR EIN ZERLEGBARES MEDIZINISCHES INSTRUMENT**
COUPLING ARRANGEMENT FOR A SEPARABLE MEDICAL INSTRUMENT
DISPOSITIF D'ACCOUPLEMENT POUR UN INSTRUMENT MÉDICAL DÉMONTABLE

(30) Priorität: 10.12.2015 DE 102015121481
(43) Veröffentlichungstag der Anmeldung: 14.06.2017
(73) Patentinhaber: Karl Storz SE & Co. KG, 78532 Tuttlingen (DE)
(72) Erfinder: Stefan, Jochen, 78532 Tuttlingen (DE); Merz, Robin, 78532 Tuttlingen (DE); Kärcher, Daniel, 78532 Tuttlingen (DE)

(56) Entgegenhaltungen:
- EP-A2- 0 513 471
- EP-B1- 1 622 521
- DE-A1- 10 328 515
- DE-U1-202009 013 504
- US-A- 5 342 391
- US-A- 5 718 714

## Beschreibung

Die vorliegende Erfindung ist auf eine Handhabungseinrichtung zur Bildung eines zerlegbaren medizinischen Instruments und auf ein zerlegbares medizinisches Instrument bezogen, wobei das zerlegbare medizinische Instrument insbesondere ein zerlegbares medizinisches Instrument für mikroinvasive Maßnahmen ist.

Wiederverwendbare medizinische Instrumente müssen nach jeder Verwendung vollständig gereinigt und sterilisiert werden. Eine möglichst weitgehende Zerlegbarkeit eines medizinischen Instruments kann eine Reinigung deutlich vereinfachen oder eine weitgehende oder vollständige Reinigung überhaupt erst ermöglichen. Ferner kann eine Zerlegbarkeit beispielsweise eine Kombination unterschiedlicher Handhabungseinrichtungen mit unterschiedlichen Schäften und unterschiedlichen Werkzeugen (beispielsweise Scheren, Zangen, Nadelhalter) ermöglichen.

Bei vielen medizinischen Instrumenten für mikroinvasive Maßnahmen sind ein distales Ende eines Schafts mit einem Werkzeug und ein proximales Ende des Schafts mit einer Handhabungseinrichtung jeweils lösbar mechanisch verbunden, wie offenbart in DE 20 2009 013504 U1, EP 1 622 521 B1, US 5 342 391 A, EP 0 513 471 A2, US 5 718 714 A, DE 103 28 515 A1. Eine Zugstange oder eine andere Kraftübertragungseinrichtung ist im Schaft angeordnet. Das distale Ende der Kraftübertragungseinrichtung ist mit dem Werkzeug mechanisch gekoppelt (und oft unlösbar verbunden). Das proximale Ende der Kraftübertragungseinrichtung wird beim Zusammensetzen des medizinischen Instruments beispielsweise durch eine Bohrung in einem ersten Bauteil der Handhabungseinrichtung hindurchgeführt und mit einem relativ zum ersten Bauteil bewegbaren zweiten Bauteil der Handhabungseinrichtung mechanisch gekoppelt.

Die fortschreitende Miniaturisierung mikroinvasiver medizinischer Instrumente hat immer kleinere Querschnitte und damit eine abnehmende mechanische Robustheit zur Folge. Insbesondere während des Zusammensetzens eines mikroinvasiven medizinischen Instruments kann beispielsweise das proximale Ende einer Kraftübertragungseinrichtung leicht verbogen werden.

In DE 10 2011 007 119 A1, in DE 10 2012 022 573 A1 und in DE 10 2012 200 073 A1 sind alternative Konzepte zur lösbaren mechanischen Kopplung eines proximalen Endes einer Kraftübertragungseinrichtung beschrieben. Diese Konzepte sind jedoch nicht für alle Anwendungen geeignet. Insbesondere erfordern sie einen Bauraum, der nicht in jedem Fall zur Verfügung steht oder zur Verfügung gestellt werden soll.

Eine Aufgabe der vorliegenden Erfindung besteht darin, eine verbesserte Handhabungseinrichtung zur Bildung eines zerlegbaren medizinischen Instruments, ein verbessertes zerlegbares medizinisches Instrument und ein verbessertes Verfahren zum Fertigen einer Handhabungseinrichtung zu schaffen.

Diese Aufgabe wird durch die Gegenstände der unabhängigen Ansprüche gelöst.

Weiterbildungen sind in den abhängigen Ansprüchen angegeben.

Ausführungsformen der vorliegenden Erfindung beruhen auf der Idee, an einer Handhabungseinrichtung eine Führungs- oder Stützeinrichtung vorzusehen, die das proximale Ende einer Kraftübertragungseinrichtung während des Kopplungsvorgangs führt bzw. stützt und damit eine Verformung des proximalen Endes der Kraftübertragungseinrichtung verhindert.

Eine Handhabungseinrichtung zur Bildung eines zerlegbaren medizinischen Instruments umfasst ein erstes Bauteil, ein zweites Bauteil, das relativ zu dem ersten Bauteil bewegbar ist, eine Kopplungseinrichtung an dem zweiten Bauteil, die ausgebildet und angeordnet ist, um innerhalb eines Arbeitsbereichs von Positionen des zweiten Bauteils mit einer korrespondierenden Kopplungseinrichtung am proximalen Ende einer Kraftübertragungseinrichtung gekoppelt zu sein, und um bei einer Löseposition des zweiten Bauteils nicht mit einer korrespondierenden Kopplungseinrichtung am proximalen Ende einer Kraftübertragungseinrichtung gekoppelt zu sein, und eine Führungseinrichtung an dem ersten Bauteil zum Führen einer korrespondierenden Kopplungseinrichtung am proximalen Ende einer Kraftübertragungseinrichtung innerhalb eines Übertragungsbereich zwischen einem Ende des Arbeitsbereichs und der Löseposition.

Die Handhabungseinrichtung ist vorgesehen und ausgebildet, um zusammen mit einer oder mehreren weiteren Komponenten ein zerstörungsfrei und reversibel zerlegbares, d. h. ohne Weiteres wieder funktionsfähig zusammensetzbares medizinisches Instrument zu bilden. Die weiteren Komponenten umfassen beispielsweise einen Schaft, eine Schere, eine Zange einen Nadelhalter oder ein anderes Werkzeug, das mit dem distalen Ende des Schafts verbunden oder verbindbar ist, und eine Kraftübertragungseinrichtung. Die Kraftübertragungseinrichtung ist innerhalb des Schafts angeordnet oder zur Anordnung innerhalb des Schafts vorgesehen. Das distale Ende der Kraftübertragungseinrichtung kann untrennbar bzw. nicht zerstörungsfrei trennbar mit dem Werkzeug mechanisch verbunden sein. Die Kraftübertragungseinrichtung ist zur Übertragung einer Kraft (parallel zur Längsrichtung der Kraftübertragungseinrichtung) und/oder eines Drehmoments vorgesehen und ausgebildet. Die Kraftübertragungseinrichtung ist insbesondere eine Zugstange aus Metall oder einem anderen Material mit geringer Dehnungselastizität. Die Kraftübertragungseinrichtung ist mit dem Werkzeug insbesondere derart gekoppelt, dass eine Verschiebung der Kraftübertragungseinrichtung nach proximal mit einem Schließen, einem Greifen oder einer schneidenden Bewegung des Werkzeugs einhergeht.

Die Handhabungseinrichtung ist insbesondere für ein bzw. zur Bildung eines zerlegbaren medizinischen Instruments für mikroinvasive Anwendungen vorgesehen und ausgebildet.

Das erste Bauteil und das zweite Bauteil sind insbesondere derart ausgebildet und angeordnet, dass sie von medizinischem Personal mittels einer Hand relativ zueinander bewegt werden können. Die Handhabungseinrichtung kann mehrere zweite Bauteile bzw. mehr als zwei relativ zueinander bewegbare Bauteile aufweisen.

Das erste Bauteil kann monolithisch oder aus zwei oder mehr Elementen zusammengesetzt sein. Das erste Bauteil ist insbesondere mit dem proximalen Ende eines Schafts mechanisch dauerhaft verbunden oder lösbar verbindbar. Das erste Bauteil ist insbesondere derart mit dem proximalen Ende eines Schafts verbunden oder verbindbar, dass der Schaft relativ zum ersten Bauteil nicht bewegt oder lediglich um seine Längsachse rotiert werden kann. Das erste Bauteil weist insbesondere ein Auge bzw. eine Grifföffnung für einen oder mehrere Finger einer menschlichen Hand auf.

Das zweite Bauteil kann monolithisch oder aus mehreren Elementen zusammengesetzt sein. Das zweite Bauteil ist relativ zum ersten Bauteil insbesondere um eine durch eine Welle oder auf andere Weise definierte Schwenkachse schwenkbar. Alternativ oder zusätzlich kann das zweite Bauteil relativ zu dem ersten Bauteil entlang eines geraden oder gekrümmten Pfads verschiebbar oder in mehreren Raumrichtungen bewegbar sein. Das zweite Bauteil weist insbesondere ein Auge bzw. eine Grifföffnung für einen oder mehrere Finger einer menschlichen Hand auf.

Der Arbeitsbereich umfasst mehrere Positionen des zweiten Bauteils relativ zum ersten Bauteil, die für die vorgesehene Verwendung der Handhabungseinrichtung als Teil eines zerlegbaren medizinischen Instruments vorgesehen sind. Durch die mechanische Kopplung des zweiten Bauteils über die Kraftübertragungseinrichtung mit einem Werkzeug geht eine Bewegung des zweiten Bauteil mit einer Bewegung des Werkzeugs einher, und jeder Position des zweiten Bauteils relativ zu dem ersten Bauteil entspricht eine Konfiguration oder Position des Werkzeugs.

Der Arbeitsbereich umfasst insbesondere eine erste extreme Position des zweiten Bauteils relativ zu dem ersten Bauteil, die mit einer ganz geschlossenen Konfiguration einer Zange oder eines Nadelhalters oder einer am Ende eines Schneidevorgangs vorliegenden Konfiguration einer Schere am distalen Ende des zerlegbaren medizinischen Instruments einhergeht. Der Arbeitsbereich umfasst insbesondere ferner eine zweite extreme Position, die mit einer ganz geöffneten Konfiguration einer Zange oder eines Nadelhalters oder einer am Anfang eines Schneidevorgangs vorliegenden Konfiguration einer Schere am distalen Ende des zerlegbaren medizinischen Instruments einhergeht. Der Arbeitsbereich umfasst insbesondere ferner alle Positionen zwischen den beiden beschriebenen extremen Positionen innerhalb des Arbeitsbereichs.

Die Löseposition des zweiten Bauteils relativ zu dem ersten Bauteil kann durch einen Anschlag bzw. zwei einander gegenüber angeordnete und bei der Löseposition einander berührende Flächen an dem ersten Bauteil und an dem zweiten Bauteil formschlüssig definiert sein. Bei der Löseposition des zweiten Bauteils relativ zu dem ersten Bauteil liegt die Handhabungseinrichtung in einer Konfiguration vor, bei der die Kopplungseinrichtung an dem zweiten Bauteil nicht mit einer korrespondierenden Kopplungseinrichtung am proximalen Ende einer Kraftübertragungseinrichtung gekoppelt ist oder sein kann. Jedoch kann die Kopplungseinrichtung an dem zweiten Bauteil mit einer korrespondierenden Kopplungseinrichtung am proximalen Ende einer Kraftübertragungseinrichtung gekoppelt werden, indem das zweite Bauteil relativ zu dem ersten Bauteil ausgehend von der Löseposition in dem Übergangsbereich zu dem Arbeitsbereich hin bewegt wird. Durch eine umgekehrte Bewegung des zweiten Bauteils von einem Ende des Arbeitsbereichs durch den Übergangsbereich hin zu der Löseposition kann die Kopplungseinrichtung an dem zweiten Bauteil von einer korrespondierenden Kopplungseinrichtung am proximalen Ende einer Kraftübertragungseinrichtung getrennt werden.

Die Kopplungseinrichtung an dem zweiten Bauteil und eine korrespondierende Kopplungseinrichtung am proximalen Ende der Kraftübertragungseinrichtung mögen ausgebildet sein, um in einem idealisierten, insbesondere reibungsfreien Fall eine klare Unterscheidung zwischen zwei unmittelbar aneinander angrenzenden Bereichen, in denen eine Kopplung vorliegt bzw. nicht vorliegt, zu ermöglichen, wobei die Grenze zwischen beiden Bereichen durch die Löseposition gebildet sein oder nahe bei der Löseposition liegen kann. In der Realität kann insbesondere Haftreibung zwischen Oberflächen, die aneinander gleiten sollen, bewirken, dass beispielsweise mit einer Bewegung des zweiten Bauteils relativ zu dem ersten Bauteil nicht eine vorgesehene Längsbewegung der Kraftübertragungseinrichtung einhergeht, sondern stattdessen das proximale Ende der Kraftübertragungseinrichtung eine Kraft orthogonal zur Längsrichtung erfährt und dabei verbogen wird. Die Führungseinrichtung kann das proximale Ende einer Kraftübertragungseinrichtung zumindest in einem Teil des Übergangsbereichs führen und stützen. Die Führungseinrichtung kann also nicht vorgesehene Kräfte, die nicht parallel zur Längsrichtung der Kraftübertragungseinrichtung sind, aufnehmen und damit eine Verformung des proximalen Endes der Kraftübertragungseinrichtung verhindern.

Bei einer Handhabungseinrichtung, wie sie hier beschrieben ist, ist die Führungseinrichtung insbesondere ausgebildet und angeordnet, um eine korrespondierende Kopplungseinrichtung am proximalen Ende einer Kraftübertragungseinrichtung innerhalb des gesamten Übergangsbereichs zu führen.

Eine Führung des proximalen Endes einer Kraftübertragungseinrichtung innerhalb des gesamten Übergangsbereichs zwischen der Löseposition und dem der Löseposition zugewandten Ende des Arbeitsbereichs kann eine Verformung und Beschädigung der Kraftübertragungseinrichtung verhindern.

Bei einer Handhabungseinrichtung, wie sie hier beschrieben ist, ist die Führungseinrichtung insbesondere ausgebildet und angeordnet, um eine korrespondierende Kopplungseinrichtung am proximalen Ende einer Kraftübertragungseinrichtung an einer Position innerhalb des Arbeitsbereichs zu führen.

Die Führungseinrichtung ist insbesondere ausgebildet und angeordnet, um eine korrespondierende Kopplungseinrichtung am proximalen Ende einer Kraftübertragungseinrichtung innerhalb eines Teils des Arbeitsbereichs oder innerhalb des gesamten Arbeitsbereichs zu führen. Eine Führung des proximalen Endes einer Kraftübertragungseinrichtung nicht nur innerhalb des Übergangsbereichs, sondern auch innerhalb eines Teils des Arbeitsbereichs oder des gesamten Arbeitsbereich kann insbesondere im Fall einer Schwenkbarkeit des zweiten Bauteils eine Verformung des proximalen Endes einer Kraftübertragungseinrichtung verhindern.

Bei einer Handhabungseinrichtung, wie sie hier beschrieben ist, umfasst die Führungseinrichtung insbesondere eine Gleitfläche zum Führen einer korrespondierenden Kopplungseinrichtung am proximalen Ende einer Kraftübertragungseinrichtung.

Die Gleitfläche ist insbesondere translationsinvariant bzw. zylindersymmetrisch bzw. parallel zur vorgesehenen Bewegungsrichtung der korrespondierenden Kopplungseinrichtung. Die Gleitfläche ist insbesondere so angeordnet, dass sie bei jeder vorgesehenen Position einer korrespondierenden Kopplungseinrichtung am proximalen Ende einer Kraftübertragungseinrichtung deren Oberfläche berührt, ohne eine Kraft auszuüben oder einen kleinen Abstand von deren Oberfläche aufweist. Ein Abstand zwischen der Gleitfläche der Führungseinrichtung und einer Oberfläche einer korrespondierenden Kopplungseinrichtung am proximalen Ende einer Kraftübertragungseinrichtung ist klein, wenn zur Berührung zwischen der Gleitführung und der Oberfläche eine Verformung der Kraftübertragungseinrichtung erforderlich ist, die keine plastische bzw. dauerhafte Verformung der Kraftübertragungseinrichtung bewirkt.

Die Gleitfläche ist insbesondere so ausgebildet und angeordnet, dass ihre mittlere Flächennormale parallel oder im Wesentlichen parallel zu einer Richtung einer Kraft, die das zweite Bauteil auf eine Kopplungseinrichtung am proximalen Ende einer Kraftübertragungseinrichtung ausüben kann, ist.

Bei einer Handhabungseinrichtung, wie sie hier beschrieben ist, ist die Gleitfläche insbesondere an einem Rand eines Stegs an dem ersten Bauteil angeordnet.

Der Steg weist insbesondere die Gestalt eines Ausschnitts aus einer ebenen Platte auf. Der Steg ist insbesondere parallel oder im Wesentlichen parallel zu einer mittleren Flächennormale der Gleitfläche.

Bei einer Handhabungseinrichtung, wie sie hier beschrieben ist, weist das zweite Bauteil insbesondere einen Schlitz auf, in dem der Steg am ersten Bauteil angeordnet ist.

Bei einer Handhabungseinrichtung, wie sie hier beschrieben ist, umfasst die Kopplungseinrichtung am zweiten Bauteil insbesondere zwei parallele und einander gegenüberliegende Nuten in zwei einander gegenüberliegenden Wänden des Schlitzes.

Die Nuten sind insbesondere so angeordnet, dass sie eine korrespondierende Kopplungseinrichtung am proximalen Ende einer Kraftübertragungseinrichtung spiel- und reibungsarm entlang eines vorbestimmten Pfads relativ zum zweiten Bauteil führen können. Die Nuten und der Pfad sind insbesondere gerade. Im Fall einer Schwenkbarkeit des zweiten Bauteils sind die Nuten beispielsweise radial zur Schwenkachse angeordnet. Die Nuten sind insbesondere so ausgebildet und angeordnet, dass bei der Löseposition des zweiten Bauteils relativ zu dem ersten Bauteil eine korrespondierende Kopplungseinrichtung am proximalen Ende einer Kraftübertragungseinrichtung an ersten Enden der Nuten angeordnet oder in erste Enden der Nuten eingeführt werden kann. Durch eine Bewegung des zweiten Bauteils relativ zu dem ersten Bauteil weg von der Löseposition und hin zum Arbeitsbereich oder innerhalb des Arbeitsbereichs kann eine Kopplungseinrichtung am proximalen Ende einer Kraftübertragungseinrichtung sowohl entlang der Führungseinrichtung relativ zu dem ersten Bauteil und gleichzeitig entlang der Nuten relativ zu dem zweiten Bauteil bewegt werden.

Bei einer Handhabungseinrichtung, wie sie hier beschrieben ist, weist die Gleitfläche insbesondere die Gestalt eines Ausschnitts aus einer Mantelfläche eines Kreiszylinders auf.

Eine Gleitfläche in Gestalt eines Ausschnitts aus einer Mantelfläche eines Kreiszylinders kann beispielsweise als Teil einer inneren Oberfläche einer Bohrung gebildet werden. Die Gleitfläche ist insbesondere die Fortsetzung einer inneren Oberfläche einer Bohrung, in die eine Kraftübertragungseinrichtung beim Zusammensetzen oder Zusammenbauen eines zerlegbaren medizinischen Instruments von distal nach proximal eingeführt und durch die eine Kopplungseinrichtung am proximalen Ende der Kraftübertragungseinrichtung hindurchgeführt werden kann.

Bei einer Handhabungseinrichtung, wie sie hier beschrieben ist, sind insbesondere die Breite des Stegs und die Breite des Schlitzes jeweils kleiner als die Breite der korrespondierenden Kopplungseinrichtung am proximalen Ende einer Kraftübertragungseinrichtung, für die die Handhabungseinrichtung vorgesehen ist.

Bei einer Handhabungseinrichtung, wie sie hier beschrieben ist, ist die Kopplungseinrichtung insbesondere zur Kopplung mit einer kugelförmigen korrespondierenden Kopplungseinrichtung am proximalen Ende einer Kraftübertragungseinrichtung ausgebildet, wobei die Breite des Stegs und die Breite des Schlitzes jeweils kleiner ist als der Durchmesser der kugelförmigen korrespondierenden Kopplungseinrichtung am proximalen Ende einer Kraftübertragungseinrichtung, für die die Handhabungseinrichtung vorgesehen ist.

Indem die Breite des Stegs kleiner ist als der Durchmesser der kugelförmigen korrespondierenden Kopplungseinrichtung, steht die kugelförmige korrespondierende Kopplungseinrichtung an einer Seite oder an beiden Seiten über den Steg über. Indem die Breite des Schlitzes kleiner ist als der Durchmesser der kugelförmigen korrespondierenden Kopplungseinrichtung kann die kugelförmige korrespondierende Kopplungseinrichtung in eine Nut an einer Innenwand des Schlitzes oder gleichzeitig in zwei einander gegenüberliegende Nuten in zwei einander gegenüberliegenden Wänden des Schlitzes eingreifen. Die kugelförmige korrespondierende Kopplungseinrichtung kann somit gleichzeitig durch den Steg bzw. die Gleitfläche an dem Steg relativ zu dem ersten Bauteil und durch die Nut oder durch die Nuten an der inneren Oberfläche des Schlitzes relativ zu dem zweiten Bauteil geführt sein.

Bei einer Handhabungseinrichtung, wie sie hier beschrieben ist, umfasst die Führungseinrichtung insbesondere zwei einander gegenüberliegende Gleitflächen, die ausgebildet und angeordnet sind, um eine korrespondierende Kopplungseinrichtung am proximalen Ende einer Kraftübertragungseinrichtung zwischen sich zu führen.

Der Querschnitt des von den zwei einander gegenüberliegenden Gleitflächen begrenzten Raums ist insbesondere nur geringfügig größer als der Querschnitt der korrespondierenden Kopplungseinrichtung am proximalen Ende der Kraftübertragungseinrichtung, für die die Handhabungseinrichtung vorgesehen ist, um eine spiel- und reibungsarme Führung zu ermöglichen. Die zwei Gleitflächen liegen einander insbesondere in einer Richtung gegenüber, die parallel zu einer Ebene, in der das zweite Bauteil liegt, ist. Insbesondere liegen die zwei Gleitflächen einander in einer Richtung gegenüber, die orthogonal zu einer Schwenkachse ist, um die das zweite Bauteil schwenkbar ist.

Bei einer Handhabungseinrichtung, wie sie hier beschrieben ist, weisen die Gleitflächen insbesondere die Gestalt zweier Ausschnitte aus einer Mantelfläche eines Kreiszylinders auf.

Insbesondere werden die Gleitflächen durch Ausschnitte bzw. Bereiche einer inneren Oberfläche einer Bohrung in dem ersten Bauteil gebildet.

Eine Handhabungseinrichtung, wie sie hier beschrieben ist, umfasst insbesondere ferner eine Schaftkupplungseinrichtung zur lösbaren mechanischen Verbindung der Handhabungseinrichtung mit einem proximalen Ende eines Schafts.

Ein zerlegbares medizinisches Instrument umfasst eine Handhabungseinrichtung, wie sie hier beschrieben ist, einen Schaft, dessen proximales Ende mit der Handhabungseinrichtung verbunden oder lösbar mechanisch verbindbar ist, und eine Kraftübertragungseinrichtung mit einer zur Kopplungseinrichtung an dem zweiten Bauteil korrespondierenden Kopplungseinrichtung am proximalen Ende der Kraftübertragungseinrichtung.

Bei einem zerlegbaren medizinischen Instrument, wie es hier beschrieben ist, ist die Kopplungseinrichtung am proximalen Ende der Kraftübertragungseinrichtung insbesondere kugelförmig.

Ein Verfahren zum Fertigen einer Handhabungseinrichtung zur Bildung eines zerlegbaren medizinischen Instruments umfasst einen Schritt des Erzeugens einer ersten Bohrung in einem ersten Bauteil, wobei die erste Bohrung zur Aufnahme eines proximalen Bereichs einer Kraftübertragungseinrichtung vorgesehen und ausgebildet ist, einen Schritt des Abtragens von Material an dem ersten Bauteil von zwei voneinander abgewandten Seiten bis zu zwei parallelen ebenen Flächen, die parallel zur Achse der ersten Bohrung sind und die innere Oberfläche der Bohrung schneiden, einen Schritt des Erzeugens einer zweiten Bohrung in einem zweiten Bauteil, wobei die zweite Bohrung zur Aufnahme einer Kopplungseinrichtung am proximalen Ende einer Kraftübertragungseinrichtung vorgesehen und ausgebildet ist, einen Schritt des Erzeugens eines Schlitzes in dem ersten Bauteil, wobei der Schlitz parallel zu einer Achse der zweiten Bohrung ist, und wobei die Breite des Schlitzes kleiner ist als der Durchmesser der zweiten Bohrung, und einen Schritt des Verbindens des ersten Bauteils und des zweiten Bauteils derart, dass das zweite Bauteil relativ zu dem ersten Bauteil bewegbar ist.

Das Verfahren ist insbesondere zum Fertigen einer Handhabungseinrichtung, wie sie hier beschrieben ist, oder einer Handhabungseinrichtung mit hier beschriebenen Merkmalen geeignet.

Die Schritte können in einer anderen Reihenfolge ausgeführt werden. Die Achse der ersten Bohrung ist insbesondere zwischen den zwei parallelen ebenen Flächen, bis zu denen Material an dem ersten Bauteil abgetragen wird, angeordnet. Die Achse der zweiten Bohrung ist insbesondere innerhalb des Schlitzes in dem zweiten Bauteil angeordnet. Die erste Bohrung, die zweite Bohrung und der Schlitz können jeweils mittels eines spanenden Verfahrens, durch Elektroerosion, Laser- oder Wasserstrahlschneiden oder auf andere Weise erzeugt werden. Auch der Abtrag von Material an dem ersten Bauteil kann mittels eines der genannten Verfahren erfolgen.

### Kurzbeschreibung der Figuren

Nachfolgend werden Ausführungsformen anhand der beigefügten Figuren näher erläutert. Es zeigen:
- Figur 1: eine schematische Darstellung eines zerlegbaren medizinischen Instruments;
- Figur 2: eine schematische Darstellung von Teilen einer Handhabungseinrichtung für das medizinische Instrument aus Figur 1;
- Figur 3: eine schematische Darstellung eines Bauteils der Handhabungseinrichtung aus Figur 2;
- Figur 4: eine schematische Schnittdarstellung des Bauteils aus Figur 3;
- Figur 5: eine schematische Schnittdarstellung eines weiteren Bauteils der Handhabungseinrichtung aus Figur 2;
- Figur 6: eine weitere schematische Schnittdarstellung des Bauteils aus Figur 5;
- Figur 7: eine weitere schematische Schnittdarstellung der Handhabungseinrichtung aus Figur 2;
- Figur 8: eine weitere schematische Darstellung der Handhabungseinrichtung aus den Figuren 2 und 7;
- Figur 9: eine weitere schematische Darstellung der Handhabungseinrichtung aus den Figuren 2, 7 und 8;
- Figur 10: eine schematische Darstellung einer weiteren Handhabungseinrichtung;
- Figur 11: ein schematisches Flussdiagramm eines Verfahrens zum Fertigen einer Handhabungseinrichtung.

### Beschreibung der Ausführungsformen

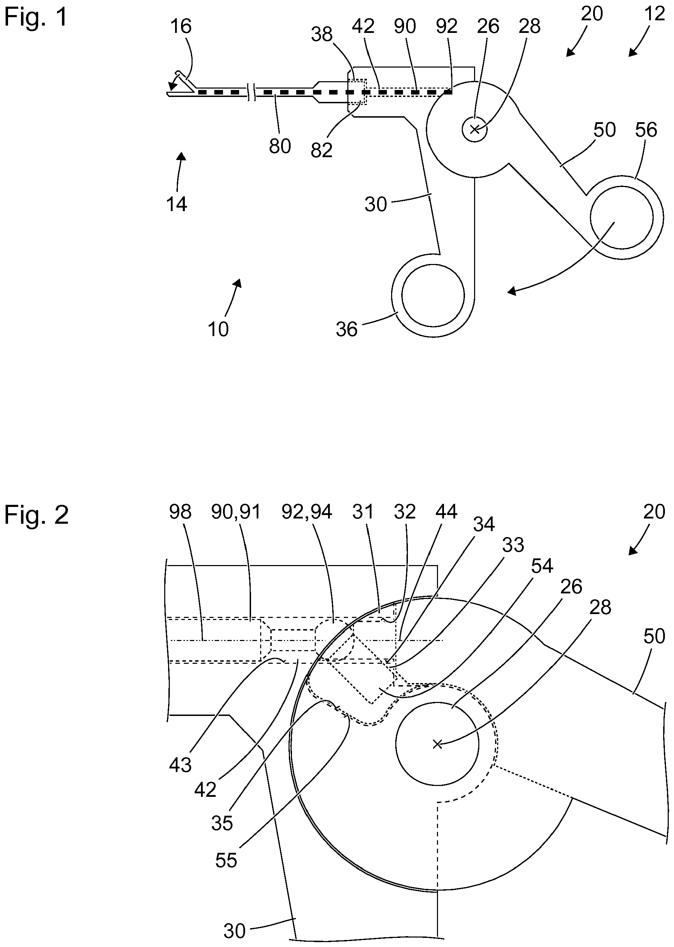

Figur 1 zeigt eine schematische Darstellung eines zerlegbaren medizinischen Instruments 10, das insbesondere für eine Verwendung im Rahmen einer mikroinvasiven Maßnahme vorgesehen und ausgebildet ist. Das proximale Ende 12 des medizinischen Instruments 10 wird durch eine Handhabungseinrichtung 20 gebildet. Das distale Ende 14 wird durch ein Werkzeug 16 gebildet. Von der Handhabungseinrichtung 20 bis zu dem Werkzeug 16 erstreckt sich ein Schaft 80. Das Werkzeug 16 ist beispielsweise eine Schere, eine Zange oder ein Nadelhalter mit zwei oder mehr Branchen, von denen mindestens eine relativ zur anderen und zum Schaft 80 bewegbar ist.

Die Handhabungseinrichtung 20 umfasst ein erstes Bauteil 30 und ein zweites Bauteil 50, die durch ein Gelenk 26 miteinander verbunden sind. Das Gelenk 26 definiert eine Schwenkachse 28 orthogonal zur Zeichenebene der Figur 1. Das Gelenk 26 ist beispielsweise eine kurze Welle, die mit dem ersten Bauteil 30 ein Gleitlager bildet und mit dem zweiten Bauteil 50 starr verbunden ist.

An dem ersten Bauteil 30 ist ein Auge bzw. eine Grifföffnung 36 für einen oder mehrere Finger einer menschlichen Hand angeordnet. An dem zweiten Bauteil 50 ist ebenfalls ein Auge bzw. eine Grifföffnung 56 für einen oder mehrere Finger einer menschlichen Hand angeordnet. Insbesondere sind das Auge 36 am ersten Bauteil 30 für den Mittelfinger, den Ringfinger oder einen anderen Finger und das Auge 56 an dem zweiten Bauteil 50 für den Daumen derselben Hand vorgesehen.

An dem ersten Bauteil 30 ist eine Ausnehmung 38 zur Aufnahme des proximalen Endes 82 des Schafts 80 vorgesehen. Die Ausnehmung 38 ist in Figur 1 vereinfacht als Sackbohrung angedeutet. Mittels einer in Figur 1 nicht dargestellten Verriegelungseinrichtung kann das proximale Ende 82 des Schafts 80 in der Ausnehmung 38 gehalten werden. Das erste Bauteil 30 weist in Verlängerung der Ausnehmung 38 eine Bohrung 42 auf. Der Querschnitt der Bohrung 42 ist kleiner als der Querschnitt der Ausnehmung 38.

Im Schaft 80 und in der Bohrung 42 ist eine Kraftübertragungseinrichtung 90 - beispielsweise eine Zugstange - angeordnet. Die Kraftübertragungseinrichtung 90 ist in Figur 1 durch eine breite gestrichelte Linie angedeutet, da sie im Inneren des Schafts 80 und des ersten Bauteils 30 angeordnet und deshalb von außen nicht sichtbar ist. Die Kraftübertragungseinrichtung 90 ist im Schaft 80 und in der Bohrung 42 in Richtung parallel zur Längsachse der Kraftübertragungseinrichtung 90 verschiebbar. Die Kraftübertragungseinrichtung 90 weist beispielsweise Metall oder ein anderes Material mit geringer Dehnelastizität auf. Die Kraftübertragungseinrichtung 90 weist insbesondere einen kleinen Querschnitt und deshalb eine hohe Biegeelastizität auf. Optional kann die Kraftübertragungseinrichtung 90 zusätzlich zur Übertragung eines Drehmoments oder einer Drehbewegung von der Handhabungseinrichtung 20 zu dem Werkzeug 16 vorgesehen und ausgebildet sein. Das proximale Ende 92 der Kraftübertragungseinrichtung 90 ist - wie anhand der Figuren 2 bis 10 dargestellt - mit dem zweiten Bauteil 50 gekoppelt, so dass eine Bewegung des zweiten Bauteils 50 mit einer Bewegung der Kraftübertragungseinrichtung 90 einhergeht. Das distale Ende der Kraftübertragungseinrichtung 90 ist mit dem Werkzeug 16 am distalen Ende 14 des medizinischen Instruments 10 derart gekoppelt, dass eine Bewegung der Kraftübertragungseinrichtung 90 beispielsweise mit einer Schwenkbewegung einer schwenkbaren Branche oder zweier schwenkbarer Branchen des Werkzeugs 16 einhergeht.

Bei dem dargestellten Beispiel sind das zweite Bauteil 50 und das proximale Ende 92 der Kraftübertragungseinrichtung 90 sowie das distale der Kraftübertragungseinrichtung 90 und das Werkzeug 16 derart gekoppelt, dass eine Schwenkbewegung des zweiten Bauteils 50 zu dem ersten Bauteil 30 hin mit einem Schließen, einer greifenden Bewegung oder einer Schneidebewegung des Werkzeugs 16 am distalen Ende 14 des medizinischen Instruments 10 einhergeht.

Das medizinische Instrument 10 ist zerstörungsfrei und reversibel zerlegbar. Insbesondere kann die Handhabungseinrichtung 20 von dem proximalen Ende 82 des Schafts 80 getrennt werden. Dazu wird ausgehend von der in Figur 1 gezeigten Situation und beispielsweise eine Drucktaste zur Entriegelung der Verbindung zwischen Handhabungseinrichtung 20 und Schaft 80 gedrückt und gleichzeitig oder danach das proximale Ende 82 des Schafts 80 aus der Ausnehmung 38 am ersten Bauteil 30 nach distal herausgezogen. Dabei wird insbesondere gleichzeitig auch die Kraftübertragungseinrichtung 90 einschließlich ihres proximalen Endes 92 aus der Bohrung 42 im ersten Bauteil 30 nach distal herausgezogen. Ferner können der Schaft 80 und das Werkzeug 16 zerstörungsfrei und reversibel lösbar mechanisch miteinander verbunden sein. Das distale Ende der Kraftübertragungseinrichtung 90 kann mit dem Werkzeug 16 dauerhaft verbunden sein.

Figur 2 zeigt eine schematische Darstellung von Teilen des zweiten Bauteils 30 und des schwenkbaren Bauteils 50 einer Handhabungseinrichtung 20 für ein medizinisches Instrument, wie es beispielsweise anhand der Figur 1 dargestellt ist. Die Zeichenebene der Figur 2 entspricht der Zeichenebene der Figur 1. Im Unterschied zu Figur 1 sind in Figur 2 nicht nur - in durchgezogenen Linien - von außen sichtbare Merkmale, sondern auch - in gestrichelten Linien - von außen nicht sichtbare Strukturen und Merkmale im Inneren der Handhabungseinrichtung 20 angedeutet. Die Handhabungseinrichtung 20 ist insbesondere spiegelsymmetrisch oder im Wesentlichen spiegelsymmetrisch zu einer Symmetrieebene, die parallel zur Zeichenebene der Figur 2 ist.

Wie die anhand der Figur 1 dargestellte Handhabungseinrichtung weist auch die in Figur 2 gezeigte Handhabungseinrichtung 20 ein erstes Bauteil 30 und ein zweites Bauteil 50 auf. Die Handhabungseinrichtung 20 umfasst ein Gelenk 26, das eine Schwenkachse 28 orthogonal zur Zeichenebene der Figur 2 definiert. In Figur 2 sind nur Bereiche des ersten Bauteils 30 und des zweiten Bauteils 50 nahe dem Gelenk 26 dargestellt. Das Gelenk 26 wird durch eine Welle oder einen kreiszylindrischen oder abschnittsweise kreiszylindrischen Bolzen, der im ersten Bauteil spiel- und reibungsarm geführt und mit dem zweiten Bauteil 50 starr verbunden ist, gebildet. Das Gelenk 26 ermöglicht eine Schwenkbewegung des zweiten Bauteils 50 um die Schwenkachse 28 relativ zum ersten Bauteil 30.

Das erste Bauteil 30 weist eine kreiszylindrische Bohrung 42 auf, in die bei der in Figur 2 gezeigten Situation bereits ein proximaler Bereich 91 einer Kraftübertragungseinrichtung 90 eingesetzt ist. Das proximale Ende 92 der Kraftübertragungseinrichtung 90 ist durch eine kugelförmige oder im Wesentlichen kugelförmige Kopplungseinrichtung 94 gebildet. Der Querschnitt der Bohrung 42 und die Querschnitte der Kraftübertragungseinrichtung 90 einschließlich der Kopplungseinrichtung 94 am proximalen Ende 92 der Kraftübertragungseinrichtung 90 sind so aufeinander abgestimmt, dass die Kraftübertragungseinrichtung 90 durch die innere Oberfläche 43 der Bohrung 42 spiel- und reibungsarm geführt und parallel zu ihrer Symmetrieachse 98 in der Bohrung 42 verschiebbar ist.

Das zweite Bauteil 50 weist an seinem dem ersten Bauteil 30 zugewandten Ende einen Schlitz auf, der sich parallel zur Zeichenebene der Figur 2 erstreckt. In dem Schlitz sind Teile des ersten Bauteils 30 angeordnet, so dass in der Blickrichtung der Figur 2 das zweite Bauteil 50 teilweise vor und teilweise hinter den in dem Schlitz angeordneten Teilen des ersten Bauteils 30 angeordnet sind.

Insbesondere sind in dem erwähnten Schlitz in dem zweiten Bauteil 50 ein erster Steg 31 und ein zweiter Steg 33 an dem ersten Bauteil 30 angeordnet. Eine erste Gleitfläche 32 an dem dem zweiten Steg 33 zugewandten geraden Rand des ersten Stegs 31 und eine zweite Gleitfläche 34 an dem dem ersten Steg 31 zugewandten geraden Rand des zweiten Stegs 33 bilden eine Führungseinrichtung für die Kopplungseinrichtung 94 am proximalen Ende 92 der Kraftübertragungseinrichtung 90 in Positionen, die proximal der in Figur 2 gezeigten Position der Kraftübertragungseinrichtung 90 liegen.

Die Gleitflächen 32, 34 weisen insbesondere die Gestalt zweier Ausschnitte aus einer Mantelfläche eines Kreiszylinders mit der Symmetrieachse 44 auf und sind deshalb jeweils konkav. Die Gleitflächen 32, 34 sind insbesondere glatte Fortsetzungen der inneren Oberfläche 43 der Bohrung 42 nach proximal. Die Symmetrieachse 44 der Gleitflächen 32, 34 ist somit identisch mit der Symmetrieachse 98 der Kraftübertragungseinrichtung 90.

An dem ersten Bauteil 30 sind Stufen 35 und an dem zweiten Bauteil 50 sind Stufen 55 vorgesehen. Insbesondere sind zwei Stufen 35 an dem ersten Bauteil 30 parallel und spiegelsymmetrisch zu einander an einer dem Betrachter zugewandten Seite und an einer vom Betrachter abgewandten Seite des ersten Bauteils 30 angeordnet. Insbesondere sind zwei Stufen 55 an dem zweiten Bauteil 50 parallel und spiegelsymmetrisch zu einander an zwei einander gegenüberliegenden Wänden des erwähnten Schlitzes angeordnet. Der zweite Steg 33 erstreckt sich insbesondere von den Stufen 35 bis zu der zweiten Gleitfläche 34.

Bei der in Figur 2 gezeigten Position des zweiten Bauteils 50 relativ zu dem ersten Bauteil 30 liegen die Stufen 55 an dem zweiten Bauteil 50 an den Stufen 35 an dem ersten Bauteil 30 an. Die Stufen 35 an dem ersten Bauteil und die Stufen 55 an dem zweiten Bauteil 50 bilden somit einen mechanischen Anschlag, der formschlüssig die in Figur 2 gezeigte Löseposition des zweiten Bauteil 50 relativ zu dem ersten Bauteil 30 mit einem maximalen Winkelabstand des zweiten Bauteils 50 vom ersten Bauteil 30 definiert.

In zwei parallelen und einander gegenüberliegenden Wänden, die den erwähnten Schlitz im zweiten Bauteil 50 begrenzen, sind zwei parallele und einander gegenüberliegende Nuten 54 vorgesehen Die Querschnitte der Nuten 54 sind so gewählt, dass sie einander gegenüberliegende Bereiche der Kopplungseinrichtung 94 aufnehmen können.

Bei der in Figur 2 dargestellten Löseposition des zweiten Bauteils 50 relativ zu dem ersten Bauteil 30 liegt die Kopplungseinrichtung 94 am proximalen Ende 92 der Kraftübertragungseinrichtung 90 an den offenen Enden der Nuten 54. Ausgehend von dieser Situation bzw. Konfiguration kann die Kraftübertragungseinrichtung 90 nach proximal geschoben werden. Dabei wird das zweite Bauteil 50 im Uhrzeigersinn um die Schwenkachse 28 geschwenkt, und die Kopplungseinrichtung 94 gleitet in die Nuten 54 hinein.

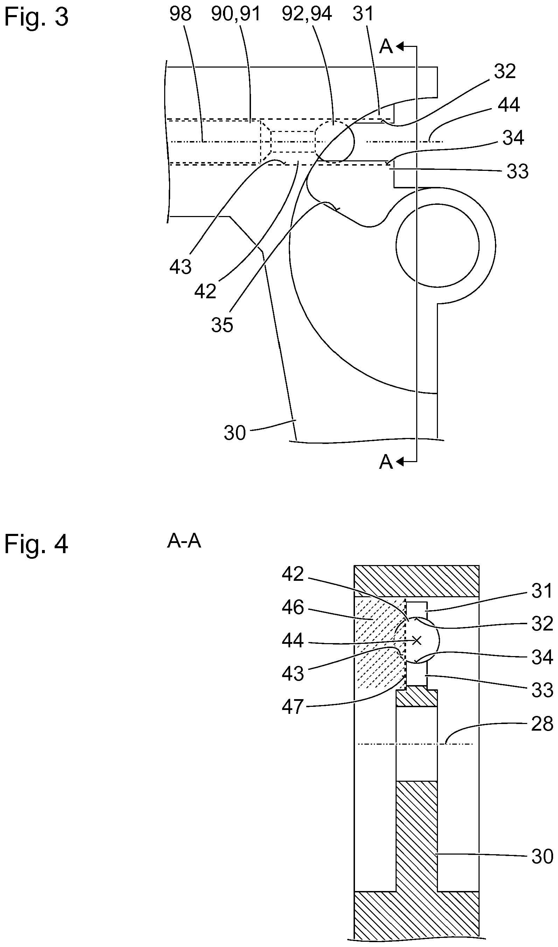

Figur 3 zeigt eine schematische Darstellung des ersten Bauteils 30 der anhand der Figur 2 dargestellten Handhabungseinrichtung 20. Die Zeichenebene der Figur 3 entspricht der Zeichenebene der Figur 2. Im Unterschied zur Darstellung in Figur 2 ist in Figur 3 nur das erste Bauteil 30, nicht jedoch das zweite Bauteil 50 dargestellt. Die Stege 31, 33 und die Stufe 35 sind deshalb sichtbar. Die Gleitflächen 32, 34 an den einander zugewandten Rändern der Stege 31, 33 sind konkav und deshalb auch in der Darstellung der Figur 3 nicht sichtbar und nur durch gestrichelte Linien angedeutet.

Figur 4 zeigt eine schematische Darstellung eines Schnitts entlang der in Figur 3 angedeuteten Ebene A-A durch das erste Bauteil 30. Die Schnittebene A-A ist orthogonal zu den Zeichenebenen der Figuren 2 und 3 und orthogonal zur Symmetrieachse 44 der Gleitflächen 32, 34.

Da die Gleitflächen 32, 34 gerade und glatte Fortsetzungen der inneren Oberfläche 43 der Bohrung 42 im ersten Bauteil 30 sind, ist die Symmetrieachse 44 der Gleitflächen 32, 34 gleichzeitig die Symmetrieachse der inneren Oberfläche 43 der Bohrung 42. Die Gleitflächen 32, 34 sind insbesondere gleichzeitig bzw. im gleichen Arbeitsgang mit der Bohrung 42 erzeugt. Der Durchmesser der Bohrung 42 entspricht im Wesentlichen dem Durchmesser der Kupplungseinrichtung 94 am proximalen Ende 92 der Kraftübertragungseinrichtung 90 (vgl. Figur 3). Der Durchmesser der Bohrung 42 ist größer als die in Richtung parallel zur Schwenkachse 28 gemessene Dicke der Stege 31, 33.

Bei der Fertigung des ersten Bauteils 30 wird insbesondere nach dem Erzeugen der Bohrung 42 als Durchgangsbohrung von zwei einander gegenüberliegenden Seiten aus symmetrisch oder im Wesentlichen symmetrisch Material abgetragen. Unter anderem wird Material in zwei zu einander spiegelsymmetrischen Raumbereichen 46, von denen einer in Figur 4 gestrichelt schraffiert dargestellt ist, abgetragen. Die beiden spiegelsymmetrischen Raumbereiche 46 werden durch spiegelsymmetrisch angeordnete ebene Flächen 47, von denen eine in Figur 4 durch eine gerade gestrichelte Linie angedeutet ist, begrenzt. Die ebenen Flächen 47, bis zu denen Material abgetragen wird, schneiden den Querschnitt der Bohrung 42. Deshalb verbleiben von der inneren Oberfläche der Bohrung 42 nach dem Abtragen von Material in den Raumbereichen 46 nur die streifenförmigen konkaven Gleitflächen 32, 34. Die ebenen Flächen 47 bilden gleichzeitig jeweils zwei von einander abgewandte Oberflächenbereiche an den Stegen 31, 33.

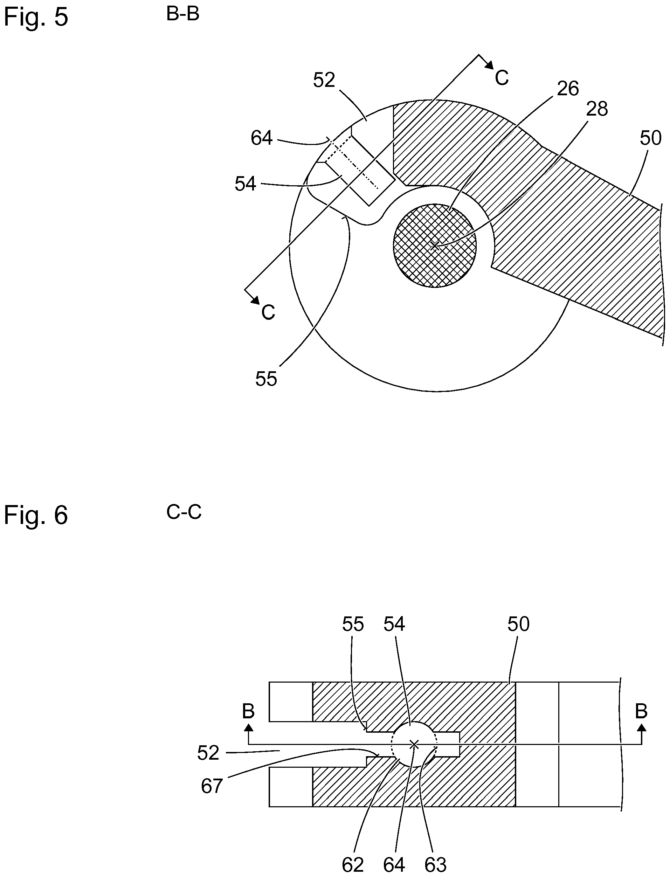

Figur 5 zeigt eine schematische Darstellung eines Schnitts durch das zweite Bauteil 50 der anhand der Figur 2 dargestellten Handhabungseinrichtung entlang einer Schnittebene B-B. Die Schnittebene B-B ist parallel zu den Zeichenebenen der Figuren 1 bis 3 und ist gleichzeitig die Symmetrieebene, zu der das zweite Bauteil 50 spiegelsymmetrisch oder im Wesentlichen spiegelsymmetrisch ist.

Die Schnittebene B-B liegt in dem erwähnten Schlitz 52 im zweiten Bauteil 50. Die Welle oder der Bolzen, die bzw. der das Gelenk 26 bildet, durchdringt den Schlitz orthogonal. Damit verbleibt eine Gestalt des Schlitzes, die im mathematischen Sinne zweifach zusammenhängend und homöomorph zu einem Ring ist.

Der Schlitz 52 weist in zwei durch die Stufe 55 getrennten Bereichen verschiedene (in Richtung parallel zur Schwenkachse 28 und orthogonal zur Schnittebene B-B gemessene) Breiten auf. In einem Bereich, in dem der Schlitz 52 eine geringere Breite aufweist, sind an den einander zugewandten und gegenüberliegenden Wänden des Schlitzes 52 je eine Nut 54 angeordnet. Die Nuten 54 sind parallel zueinander und weisen innere Oberflächen auf, die im Wesentlichen die Gestalt zweier Ausschnitte der Mantelfläche eines Kreiszylinders mit der Symmetrieachse 64 aufweisen. Abweichend davon sind an den radial äußeren bzw.
von dem Gelenk 26 abgewandten Enden der Nuten 54 Fasen bzw. trichterförmige Eingänge vorgesehen.

Figur 6 zeigt eine schematische Darstellung eines Schnitts entlang der in Figur 5 angedeuteten Schnittebene C-C durch das anhand der Figuren 2 und 5 dargestellte zweite Bauteil 50. Die Schnittebene C-C ist orthogonal zur Schnittebene B-B der Figur 5, parallel zur Schwenkachse 28 und orthogonal zur Symmetrieachse 64 der Nuten 54. Die Lage der Schnittebene B-B der Figur 5, zu der das zweite Bauteil 50 spiegelsymmetrisch ist, ist in Figur 6 angedeutet.

In Figur 6 ist erkennbar, dass der Schlitz 52 zwei Bereiche unterschiedlicher Breite aufweist, zwischen denen die Stufen 55 angeordnet sind. Die Flächennormalen der Stufen 55 sind parallel zur Schnittebene B-B der Figur 5 und orthogonal zur Schwenkachse 28 (vgl. Figuren 2 und 5).

Die Nuten 54 werden insbesondere gleichzeitig und vor der Erzeugung des Schlitzes 52 durch Erstellen einer Bohrung 62, deren Symmetrieachse der Symmetrieachse 64 der Nuten 54 entspricht, erzeugt. Die Symmetrieachse 64 der Bohrung 62 und der Nuten 54 liegt in der Symmetrieebene des zweiten Bauteils 50 bzw. in der Schnittebene B-B der Figur 5. Der Schlitz 52 wird in der Umgebung der Nuten 54 durch zwei parallele und einander gegenüberliegende Oberflächen 67, deren Abstand geringer ist als der Durchmesser der Bohrung 62, begrenzt. Durch das Erzeugen des Schlitzes 52 bleiben von der inneren Oberfläche 63 der Bohrung 62 nur die konkaven Oberflächen der Nuten 54 übrig.

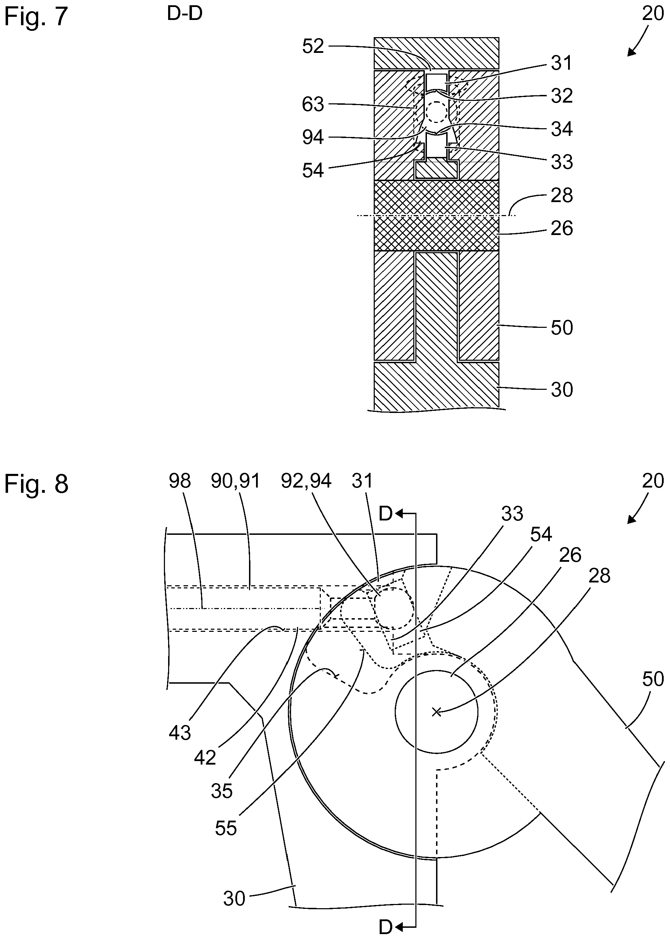

Figur 7 zeigt eine schematische Darstellung eines Schnitts entlang einer Schnittebene D-D durch die anhand der Figuren 2 bis 6 dargestellte Handhabungseinrichtung 20. Die Schnittebene D-D entspricht der Schnittebene A-A der Figur 4. In Figur 7 ist eine Konfiguration bzw. Winkelposition des zweiten Bauteils 50 relativ zu dem ersten Bauteil 30 gezeigt, die sich von der anhand der Figur 2 dargestellten unterscheidet, und die anhand der Figur 8 beschrieben ist.

Die Nuten 54 sind durch die Schnittebene D-D angeschnitten. In dem dargestellten Schnitt unsichtbare Konturen der konkaven Oberflächen der Nuten 54 sind in Figur 7 durch gestrichelte Linien angedeutet.

Die Breiten der Stege 31, 33 entsprechen weitgehend der Breite der Nut 52 in der Umgebung der Nuten 54, so dass die Stege 31, 33 am ersten Bauteil 30 reibungsarm im Schlitz 52 im zweiten Bauteil 50 geführt sind. Die Kopplungseinrichtung 94 am proximalen Ende der Kraftübertragungseinrichtung ist breiter als die Stege 31, 33 an dem ersten Bauteil 30 und der Schlitz 52 in dem zweiten Bauteil 50. Die Kopplungseinrichtung 94 greift in die Nuten 54 in dem zweiten Bauteil 50 ein. Die Gleitflächen 32, 34 führen und stützen die Kopplungseinrichtung 94 indem sie eine Auslenkung der Kopplungseinrichtung 94 in einer Richtung in der Schnittebene D-D formschlüssig unterbinden.

Figur 8 zeigt eine weitere schematische Darstellung der anhand der Figuren 2 bis 7 dargestellten Handhabungseinrichtung 20. Die Zeichenebene und die Art der Darstellung entsprechen denjenigen der Figur 2. In Figur 8 ist jedoch die Situation bzw. Konfiguration gezeigt, die auch in Figur 7 gezeigt ist, und die sich von der in Figur 2 gezeigten unterscheidet. Die Schnittebene D-D der Figur 7 ist in Figur 8 angedeutet.

Bei der in Figur 8 gezeigten Konfiguration ist das zweite Bauteil 50 ausgehend von der in Figur 2 gezeigten Löseposition um einen vorbestimmten Winkel hin zu dem ersten Bauteil 30 geschwenkt. Entsprechend ist die Stufe 55 am schwenkbaren Bauteil 50 von der Stufe 35 am ersten Bauteil 30 beabstandet. Die in Figur 8 gezeigte Position des zweiten Bauteils 50 liegt beispielsweise an dem der in Figur 2 gezeigten Löseposition zugewandten Ende eines Arbeitsbereichs von Positionen. Der Arbeitsbereich wird durch alle Positionen, die für die vorgesehene Verwendung der Handhabungseinrichtung 20 als Bestandteil eines medizinischen Instruments vorgesehen sind, gebildet. Die vorgesehene Verwendung ist die Verwendung bei einer medizinischen Maßnahme und umfasst nicht das Zerlegen, Reinigen, Sterilisieren, Zusammensetzen und Lagern des medizinischen Instruments zwischen zwei Verwendungen bei medizinischen Maßnahmen.

Die Kopplungseinrichtung 94 am proximalen Ende 92 der Kraftübertragungseinrichtung 90 ist vollständig in die Nuten 54 im zweiten Bauteil 50 eingeführt und befindet sich gleichzeitig in der äußerst proximalen Position, in der sie noch durch die Gleitflächen an den Stegen 31, 33 geführt ist. Innerhalb des gesamten Übergangsbereichs zwischen der in Figur 2 dargestellten Löseposition und der in Figur 8 dargestellten Position an einem Ende des Arbeitsbereichs ist die Kopplungseinrichtung 94 am proximalen Ende 92 der Kraftübertragungseinrichtung 90 durch die Gleitflächen 32, 34 geführt und gestützt.

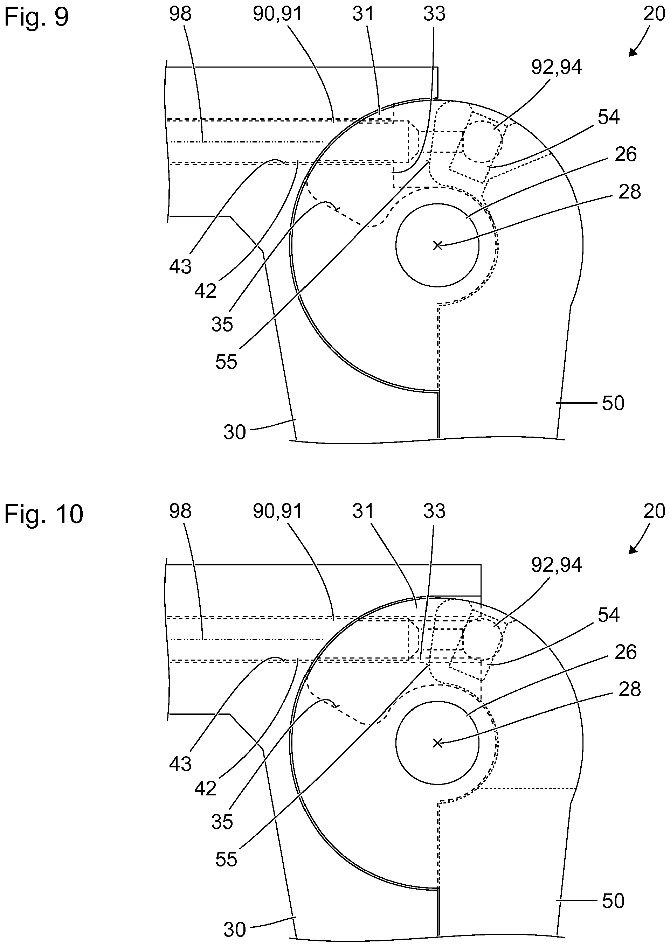

Figur 9 zeigt eine weitere schematische Darstellung der anhand der Figuren 2 bis 8 dargestellten Handhabungseinrichtung 20. Die Zeichenebene und die Art der Darstellung entsprechen denjenigen der Figuren 2 und 8. Die in Figur 9 gezeigte Konfiguration bzw. Situation unterscheidet sich jedoch von den in den Figuren 2 und 8 dargestellten.

In Figur 9 ist eine Konfiguration bzw. Situation dargestellt, bei der das zweite Bauteil 50 ganz an das erste Bauteil 30 heran geschwenkt ist. Diese Position des zweiten Bauteils 50 liegt an dem zu der in Figur 8 gezeigten Position entgegengesetzten Ende des Arbeitsbereichs. Die Kopplungseinrichtung 94 am proximalen Ende 92 der Kraftübertragungseinrichtung 90 ist in den Nuten 54 angeordnet. Innerhalb des gesamten Arbeitsbereichs, der sich von der in Figur 9 gezeigten Position bis zu der in Figur 8 gezeigten Position des zweiten Bauteils 50 (oder noch darüber hinaus) erstreckt, greift die Kopplungseinrichtung 94 am proximalen Ende 92 der Kraftübertragungseinrichtung 90 in die Nuten 54 am zweiten Bauteil 50 ein. Dieser Eingriff bildet eine formschlüssige Kopplung der Kraftübertragungseinrichtung 90 mit dem zweiten Bauteil 50, so dass eine Schwenkbewegung des zweiten Bauteils 50 um die Schwenkachse 28 mit einer Translationsbewegung der Kraftübertragungseinrichtung 90 in Richtung parallel zu ihrer Symmetrieachse 98 einhergeht.

Durch in den Figuren 2 bis 9 nicht dargestellte Einrichtungen kann sichergestellt sein, dass beispielsweise nur bei gleichzeitigem Betätigen eines Entriegelungselements die Kraftübertragungseinrichtung 90 über die in Figur 8 gezeigte Position hinaus nach distal verschoben und damit das zweite Bauteil 50 über die in Figur 8 gezeigte Winkelposition hinaus bis zu der in Figur 2 gezeigten Löseposition geschwenkt werden kann. In der in Figur 2 gezeigten Löseposition gerät die Kopplungseinrichtung 94 am proximalen Ende 92 der Kraftübertragungseinrichtung 90 außer Eingriff in die Nuten 54 im zweiten Bauteil 50, so dass die Kraftübertragungseinrichtung 90 (insbesondere zusammen mit dem Schaft 80 - vgl. Figur 1) nach distal von der Handhabungseinrichtung 20 getrennt werden kann. Die bei der in Figur 2 gezeigten Löseposition des zweiten Bauteils 50 vorliegende Position der Kraftübertragungseinrichtung 90 kann einer Überoffenstellung des Werkzeugs 16 am distalen Ende 14 des medizinischen Instruments entsprechen (vgl. Figur 1).

Figur 10 zeigt eine schematische Darstellung einer weiteren Handhabungseinrichtung, die in einigen Merkmalen, Eigenschaften und Funktionen der anhand der Figuren 2 bis 9 dargestellten Handhabungseinrichtung 20 ähnelt. Die Zeichenebene und die Art der Darstellung entsprechen denjenigen der Figuren 2, 8 und 9.

Die in Figur 10 gezeigte Handhabungseinrichtung 20 unterscheidet sich von der anhand der Figuren 2 bis 9 dargestellten Handhabungseinrichtung insbesondere dadurch, dass die Stege 31 ,33 deutlich weiter nach proximal reichen. Bei dem in Figur 10 dargestellten Beispiel reichen die Stege 31, 33 und damit auch die Gleitflächen 32, 34 an den einander zugewandten Kanten der Stege 31, 33 so weit nach proximal, dass die Kopplungseinrichtung 94 am proximalen Ende 92 der Kraftübertragungseinrichtung 90 im gesamten Arbeitsbereich, d. h. auch in der in Figur 10 gezeigten Position des zweiten Bauteils 50 von den Gleitflächen an den Stegen 31, 33 geführt ist. Eine Verformung der Kraftübertragungseinrichtung 90 ist deshalb nicht nur im Übergangsbereich zwischen der in Figur 2 gezeigten Löseposition und der in Figur 8 gezeigten Position am Rand des Arbeitsbereichs, sondern auch im gesamten Arbeitsbereich zwischen der in Figur 8 gezeigten Position und der in den Figuren 9 und 10 gezeigten Position ausgeschlossen.

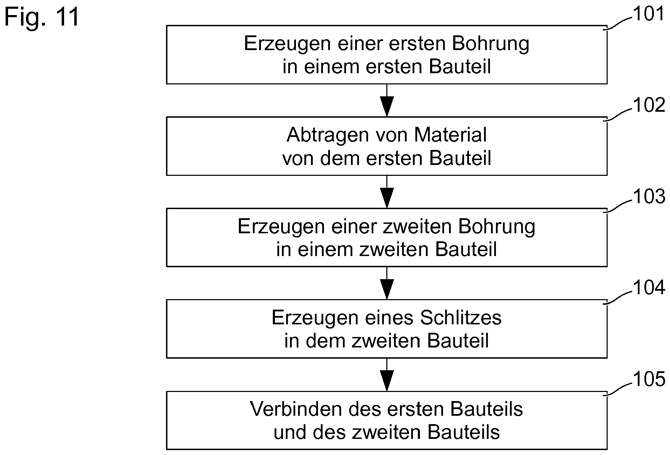

Figur 11 zeigt ein schematisches Flussdiagramm mehrerer Verfahrensschritte eines Verfahrens zum Fertigen einer Handhabungseinrichtung. Obwohl mittels eines Verfahrens mit den anhand der Figur 11 dargestellten Schritten auch eine Handhabungseinrichtung gefertigt werden kann, die andere Merkmale, Eigenschaften oder Funktionen als die anhand der Figuren 2 bis 10 dargestellten Handhabungseinrichtungen aufweist, werden nachfolgend beispielhaft Bezugszeichen aus den Figuren 1 bis 10 verwendet.

Bei einem ersten Schritt 101 wird eine erste Bohrung 42 in einem ersten Bauteil 30 bzw. in einem Werkstück, aus dem das erste Bauteil 30 entsteht, erzeugt. Gleichzeitig bzw. beim selben Verfahrensschritt oder bevor oder danach kann eine Ausnehmung 38 für ein proximales Ende 82 eines Schafts 80 erzeugt werden. Die erste Bohrung 42 weist einen Querschnitt auf, der nur unwesentlich größer ist als der Querschnitt einer Kopplungseinrichtung 94 am proximalen Ende 92 einer Kraftübertragungseinrichtung 90, für die die Handhabungseinrichtung 20 vorgesehen ist.

Bei einem zweiten Schritt 102 wird Material vom ersten Bauteil 30 abgetragen. Insbesondere wird in zwei symmetrisch zueinander und symmetrisch zur ersten Bohrung 42 angeordneten Raumbereichen 46 Material bis zu zwei parallelen ebenen Flächen 47 abgetragen. Die Achse 44 der ersten Bohrung 42 ist insbesondere parallel zu den ebenen Flächen 47 und in der Mitte zwischen den ebenen Flächen 47 angeordnet. Der Abstand der ebenen Flächen 47, bis zu denen Material abgetragen wird, ist kleiner als der Durchmesser der ersten Bohrung 42, so dass die ebenen Flächen 47 den Querschnitt der ersten Bohrung 42 schneiden. Deshalb werden beim Abtragen 102 des Materials Teile der inneren Oberfläche 43 der ersten Bohrung 42 entfernt. Verbleibende Bereiche der inneren Oberfläche 43 der ersten Bohrung 42 bilden Gleitflächen 32, 34 an parallelen und einander gegenüberliegenden Rändern zweier Stege 31, 33, die durch die ebenen Flächen 47 begrenzt werden.

Bei einem dritten Schritt 103 wird eine zweite Bohrung 62 in einem zweiten Bauteil 50 bzw. in einem Werkstück, aus dem das zweite Bauteil 50 entsteht, erzeugt. Der Querschnitt der zweiten Bohrung ist etwas größer als der Querschnitt der Kopplungseinrichtung 94 am proximalen Ende 92 der Kraftübertragungseinrichtung 90, für die die Handhabungseinrichtung 20 vorgesehen ist. Beispielsweise entspricht der Querschnitt der zweiten Bohrung 62 in dem zweiten Bauteil 50 dem Querschnitt der ersten Bohrung 42 in dem ersten Bauteil 30.

Bei einem vierten Schritt 104 wird ein Schlitz 52 in dem zweiten Bauteil 50 erzeugt. Der Schlitz 52 wird durch zwei parallele ebene Oberflächen 67 begrenzt, die parallel zur Achse 64 der zweiten Bohrung 62 sind. Die Achse 64 der zweiten Bohrung 62 ist in der Mitte zwischen den ebenen Oberflächenbereichen 67 des Schlitzes 52 angeordnet. Der Abstand der ebenen Oberflächenbereiche 67 des Schlitzes 52 ist etwas größer als der Abstand der ebenen Flächen 47 und die Breite der Stege 31, 33 am ersten Bauteil 30. Beim Erzeugen 104 des Schlitzes 52 werden Teile der inneren Oberfläche der zweiten Bohrung 62 im zweiten Bauteil 50 abgetragen. Verbleibende Bereiche der inneren Oberfläche 63 der zweiten Bohrung 62 bilden innere Oberflächen zweier Nuten 54 in den im Übrigen ebenen Oberflächenbereichen 67 des Schlitzes 52.

Bei einem fünften Schritt 105 werden das erste Bauteil 30 und das zweite Bauteil 50 durch ein Gelenk so verbunden, dass das zweite Bauteil 50 relativ zu dem ersten Bauteil 30 um eine Schwenkachse 28 schwenkbar ist, die orthogonal zur Achse 44 der ersten Bohrung 42 in dem ersten Bauteil 30 und orthogonal zur Achse 64 der zweiten Bohrung 62 in dem zweiten Bauteil 50 und damit sowohl orthogonal zu der Symmetrieachse der Gleitflächen 32, 34 am ersten Bauteil 30 als auch orthogonal zur Symmetrieachse der Nuten 54 am zweiten Bauteil 50 ist.

Die Reihenfolge der Schritte kann von der dargestellten abweichen. Insbesondere können der dritte Schritt 103 und/oder der vierte Schritt 104 vor dem ersten Schritt 101 und/oder vor dem zweiten Schritt 102 ausgeführt werden. Ferner können der zweite Schritt 102 vor dem ersten Schritt 101 und der vierte Schritt 104 vor dem dritten Schritt 103 ausgeführt werden.

### Bezugszeichen

- 10: medizinisches Instrument
- 12: proximales Ende des medizinischen Instruments 10
- 14: distales Ende des medizinischen Instruments 10
- 16: Werkzeug des medizinischen Instruments 10
- 20: Handhabungseinrichtung des medizinischen Instruments 10
- 26: Gelenk zwischen dem ersten Bauteil 30 und dem zweiten Bauteil 50
- 28: Schwenkachse des Gelenks 26
- 30: erstes Bauteil der Handhabungseinrichtung 20
- 31: erster Steg an dem ersten Bauteil 30
- 32: erste Gleitfläche an einem Rand des ersten Stegs 31
- 33: zweiter Steg an dem ersten Bauteil 30
- 34: zweite Gleitfläche an einem Rand des zweiten Stegs 33
- 35: Stufe an dem ersten Bauteil 30 als Anschlag
- 36: Auge bzw. Grifföffnung an dem ersten Bauteil 30
- 38: Ausnehmung zur Aufnahme des proximalen Ends 82 des Schafts 80
- 42: erste Bohrung in dem ersten Bauteil 30
- 43: innere Oberfläche der ersten Bohrung 42 in dem ersten Bauteil 30
- 44: Symmetrieachse der Gleitflächen 32, 34 und der ersten Bohrung 42 im ersten Bauteil 30
- 46: Raumbereich an dem ersten Bauteil 30, in dem Material abgetragen ist
- 47: an den Raumbereich 46 angrenzende ebene Fläche
- 50: zweites Bauteil der Handhabungseinrichtung 20
- 52: Schlitz in dem zweiten Bauteil 50
- 54: Nut in dem zweiten Bauteil 50
- 55: Stufe an dem zweiten Bauteil 50 als Anschlag
- 56: Auge bzw. Grifföffnung an dem zweiten Bauteil 50
- 62: zweite Bohrung in dem zweiten Bauteil 50
- 63: innere Oberfläche der zweiten Bohrung 62 in dem zweiten Bauteil 50
- 64: Symmetrieachse der Nuten 54 und der zweiten Bohrung 62 in dem zweiten Bauteil 50
- 67: den Schlitz 52 in dem zweiten Bauteil 50 begrenzende ebene Fläche
- 80: Schaft des medizinischen Instruments 10
- 82: proximales Ende des Schafts 80
- 90: Kraftübertragungseinrichtung des medizinischen Instruments 10
- 91: proximaler Bereich der Kraftübertragungseinrichtung 90
- 92: proximales Ende der Kraftübertragungseinrichtung 90
- 94: Kopplungseinrichtung am proximalen Ende 92 der Kraftübertragungseinrichtung 90
- 98: Symmetrieachse der Kraftübertragungseinrichtung 90
- 101: erster Schritt (Erzeugen einer ersten Bohrung im ersten Bauteil 30)
- 102: zweiter Schritt (Abtragen von Material am ersten Bauteil 30)
- 103: dritter Schritt (Erzeugen einer zweiten Bohrung in dem zweiten Bauteil 50)
- 104: vierter Schritt (Erzeugen eines Schlitzes in dem zweiten Bauteil 50)
- 105: fünfter Schritt (Verbinden von erstem Bauteil 30 und zweitem Bauteil 50)

## Patentansprüche

1. Handhabungseinrichtung (20) zur Bildung eines zerlegbaren medizinischen Instruments (10), mit:
einem ersten Bauteil (30);
einem zweiten Bauteil (50), das relativ zu dem ersten Bauteil (30) bewegbar ist;
einer Kopplungseinrichtung (54) an dem zweiten Bauteil (50), wobei die Kopplungseinrichtung (54) ausgebildet und angeordnet ist, um innerhalb eines Arbeitsbereichs von Positionen des zweiten Bauteils (50) mit einer korrespondierenden Kopplungseinrichtung (94) am proximalen Ende (92) einer Kraftübertragungseinrichtung (90) gekoppelt zu sein, und um bei einer Löseposition des zweiten Bauteils (50) nicht mit einer korrespondierenden Kopplungseinrichtung (94) am proximalen Ende (92) einer Kraftübertragungseinrichtung (90) gekoppelt zu sein;
einer Führungseinrichtung (32, 34) an dem ersten Bauteil (30) zum Führen einer korrespondierenden Kopplungseinrichtung (94) am proximalen Ende (92) einer Kraftübertragungseinrichtung (90) innerhalb eines Übergangsbereichs zwischen einem Ende des Arbeitsbereichs und der Löseposition.

2. Handhabungseinrichtung (20) nach einem der vorangehenden Ansprüche, bei der die Führungseinrichtung (32, 34) ausgebildet und angeordnet ist, um eine korrespondierende Kopplungseinrichtung (94) am proximalen Ende (92) einer Kraftübertragungseinrichtung (90) innerhalb des gesamten Übergangsbereichs zu führen.

3. Handhabungseinrichtung (20) nach einem der vorangehenden Ansprüche, bei der die Führungseinrichtung (32, 34) ausgebildet und angeordnet ist, um eine korrespondierende Kopplungseinrichtung (94) am proximalen Ende (92) einer Kraftübertragungseinrichtung (90) an einer Position innerhalb des Arbeitsbereichs zu führen.

4. Handhabungseinrichtung (20) nach einem der vorangehenden Ansprüche, bei der die Führungseinrichtung eine Gleitfläche (32, 34) zum Führen einer korrespondierenden Kopplungseinrichtung (94) am proximalen Ende (92) einer Kraftübertragungseinrichtung (90) umfasst.

5. Handhabungseinrichtung (20) nach dem vorangehenden Anspruch, bei der die Gleitfläche (32, 34) an einem Rand eines Stegs (31, 33) an dem ersten Bauteil (30) angeordnet ist.

6. Handhabungseinrichtung (20) nach dem vorangehenden Anspruch, bei der das zweite Bauteil (50) einen Schlitz (52) aufweist, in dem der Steg (31, 33) am ersten Bauteil (30) angeordnet ist.

7. Handhabungseinrichtung (20) nach dem vorangehenden Anspruch, bei der die Kopplungseinrichtung (54) an dem zweiten Bauteil (50) zwei parallele und einander gegenüberliegende Nuten (54) in zwei einander gegenüberliegenden Wänden (67) des Schlitzes (52) umfasst.

8. Handhabungseinrichtung (20) nach einem der Ansprüche 4 bis 7, bei der die Gleitfläche (32, 34) die Gestalt eines Ausschnitts aus einer Mantelfläche eines Kreiszylinders aufweist.

9. Handhabungseinrichtung (20) nach dem vorangehenden Anspruch, bei der
die Kopplungseinrichtung (54) zur Kopplung mit einer kugelförmigen korrespondierenden Kopplungseinrichtung (94) am proximalen Ende (92) einer Kraftübertragungseinrichtung (90) ausgebildet ist,
die Breite des Stegs (31, 33) und die Breite des Schlitzes (52) jeweils kleiner ist als der Durchmesser der kugelförmigen korrespondierenden Kopplungseinrichtung (94) am proximalen Ende (92) einer Kraftübertragungseinrichtung (90), für die die Handhabungseinrichtung (20) vorgesehen ist.

10. Handhabungseinrichtung (20) nach einem der vorangehenden Ansprüche, bei der die Führungseinrichtung zwei einander gegenüberliegende Gleitfläche (32, 34), die ausgebildet und angeordnet sind, um eine korrespondierende Kopplungseinrichtung (94) am proximalen Ende (92) einer Kraftübertragungseinrichtung (90) zwischen sich zu führen, umfasst.

11. Handhabungseinrichtung (20) nach dem vorangehenden Anspruch, bei der die Gleitflächen (32, 34) die Gestalt zweier Ausschnitte aus einer Mantelfläche eines Kreiszylinders aufweisen.

12. Zerlegbares medizinisches Instrument (10) mit:
einer Handhabungseinrichtung (20) nach einem der vorangehenden Ansprüche;
einem Schaft (80), dessen proximales Ende (82) mit der Handhabungseinrichtung (20) verbunden oder lösbar mechanisch verbindbar ist;
einer Kraftübertragungseinrichtung (90) mit einer zur Kopplungseinrichtung (54) an dem zweiten Bauteil (50) korrespondierenden Kopplungseinrichtung (94) am proximalen Ende (92) der Kraftübertragungseinrichtung (90).

13. Verfahren zum Fertigen einer Handhabungseinrichtung (20) zur Bildung eines zerlegbaren medizinischen Instruments (10) nach Anspruch 12, mit folgenden Schritten:
Erzeugen (101) einer ersten Bohrung (42) in einem ersten Bauteil (30), wobei die erste Bohrung (42) zur Aufnahme eines proximalen Bereichs (91) einer Kraftübertragungseinrichtung (90) vorgesehen und ausgebildet ist;
Abtragen (102) von Material an dem ersten Bauteil (30) von zwei von einander abgewandten Seiten bis zu zwei parallelen ebenen Flächen (47), die parallel zur Achse (44) der ersten Bohrung (42) sind und die innere Oberfläche (43) der ersten Bohrung (42) schneiden;
Erzeugen (103) einer zweiten Bohrung (62) in einem zweiten Bauteil (50), wobei die zweite Bohrung (62) zur Aufnahme einer Kopplungseinrichtung (94) am proximalen Ende (92) einer Kraftübertragungseinrichtung (90) vorgesehen und ausgebildet ist;
Erzeugen (104) eines Schlitzes (52) in dem zweiten Bauteil (50), wobei der Schlitz (52) parallel zu einer Achse (64) der zweiten Bohrung (62) ist und wobei die Breite des Schlitzes (52) kleiner ist als der Durchmesser der zweiten Bohrung (62);
Verbinden (105) des ersten Bauteils (30) und des zweiten Bauteils (50) derart, dass das zweite Bauteil (50) relativ zum ersten Bauteil (30) bewegbar ist.

## Claims

1. Handling device (20) for the formation of a disassemblable medical instrument (10), having:
a first component (30);
a second component (50), which can be moved relative to the first component (30);
a coupling device (54) on the second component (50), wherein the coupling device (54) is designed and arranged in order to be coupled to a corresponding coupling device (94) at the proximal end (92) of a force transmission device (90) within a working range of positions of the second component (50) and in order not to be coupled to a corresponding coupling device (94) at the proximal end (92) of a force transmission device (90) in a release position of the second component (50);
a guiding device (32, 34) on the first component (30) for guiding a corresponding coupling device (94) at the proximal end (92) of a force transmission device (90) within a transitional range between one end of the working range and the release position.

2. Handling device (20) according to one of the preceding claims, in which the guiding device (32, 34) is designed and arranged in order to guide a corresponding coupling device (94) at the proximal end (92) of a force transmission device (90) within the entire transitional range.

3. Handling device (20) according to one of the preceding claims, in which the guiding device (32, 34) is designed and arranged in order to guide a corresponding coupling device (94) at the proximal end (92) of a force transmission device (90) at one position within the working range.

4. Handling device (20) according to one of the preceding claims, in which the guiding device comprises a sliding surface (32, 34) for guiding a corresponding coupling device (94) at the proximal end (92) of a force transmission device (90).

5. Handling device (20) according to the preceding claim, in which the sliding surface (32, 34) is arranged at one edge of a web (31, 33) on the first component (30).

6. Handling device (20) according to the preceding claim, in which the second component (50) has a slot (52), in which the web (31, 33) on the first component (30) is arranged.

7. Handling device (20) according to the preceding claim, in which the coupling device (94) on the second component (50) comprises two parallel and mutually opposite grooves (54) in two mutually opposite walls (67) of the slot (52).

8. Handling device (20) according to one of Claims 4 to 7, in which the sliding surface (32, 34) has the form of a segment of a lateral surface of a circular cylinder.

9. Handling device (20) according to the preceding claim, in which
the coupling device (54) is designed for coupling to a spherical corresponding coupling device (94) at the proximal end (92) of a force transmission device (90),
the width of the web (31, 33) and the width of the slot (52) are each less than the diameter of the spherical corresponding coupling device (94) at the proximal end (92) of a force transmission device (90) for which the handling device (20) is provided.

10. Handling device (20) according to one of the preceding claims, in which the guiding device comprises two mutually opposite sliding surfaces (32, 34), which are designed and arranged in order to guide between them a corresponding coupling device (94) at the proximal end (92) of a force transmission device (90).

11. Handling device (20) according to the preceding claim, in which the sliding surfaces (32, 34) have the form of two segments of a lateral surface of a circular cylinder.

12. Disassemblable medical instrument (10) having:
a handling device (20) according to one of the preceding claims;
a shank (80), the proximal end (82) of which is connected to or can be connected mechanically in a releasable manner to the handling device (20);
a force transmission device (90) having a coupling device (94), corresponding to the coupling device (54) on the second component (50), at the proximal end (92) of the force transmission device (90).

13. Method for producing a handling device (20) for the formation of a disassemblable medical instrument (10) according to Claim 12, comprising the following steps:
producing (101) a first bore (42) in a first component (30), wherein the first bore (42) is provided and designed to receive a proximal region (91) of a force transmission device (90);
removing (102) material from the first component (30) on two opposite sides as far as two parallel flat surfaces (47), which are parallel to the axis (44) of the first bore (42) and intersect the inner surface (43) of the first bore (42);
producing (103) a second bore (62) in a second component (50), wherein the second bore (62) is provided and designed to receive a coupling device (94) at the proximal end (92) of a force transmission device (90) ;
producing (104) a slot (52) in the second component (50), wherein the slot (52) is parallel to an axis (64) of the second bore (62) and wherein the width of the slot (52) is less than the diameter of the second bore (62) ;
connecting (105) the first component (30) and the second component (50) in such a way that the second component (50) can be moved relative to the first component (30).

## Revendications

1. Dispositif de manipulation (20) pour former un instrument médical démontable (10), comprenant :
un premier composant (30) ;
un deuxième composant (50) qui peut être déplacé par rapport au premier composant (30) ;
un dispositif d'accouplement (54) sur le deuxième composant (50), le dispositif d'accouplement (54) étant réalisé et disposé de manière à être accouplé, à l'intérieur d'une région de travail de positions du deuxième composant (50), à un dispositif d'accouplement correspondant (94) à l'extrémité proximale (92) d'un dispositif de transfert de force (90), et de manière à ne pas être accouplé, dans une position de desserrage du deuxième composant (50), à un dispositif d'accouplement correspondant (94) à l'extrémité proximale (92) d'un dispositif de transfert de force (90) ;
un dispositif de guidage (32, 34) au niveau du premier composant (30) pour le guidage d'un dispositif d'accouplement correspondant (94) à l'extrémité proximale (92) d'un dispositif de transfert de force (90) à l'intérieur d'une région de transition entre une extrémité de la région de travail et la position de desserrage.

2. Dispositif de manipulation (20) selon l'une quelconque des revendications précédentes, dans lequel le dispositif de guidage (32, 34) est réalisé et disposé de manière à guider un dispositif d'accouplement correspondant (94) à l'extrémité proximale (92) d'un dispositif de transfert de force (90) à l'intérieur de toute la région de transition.

3. Dispositif de manipulation (20) selon l'une quelconque des revendications précédentes, dans lequel le dispositif de guidage (32, 34) est réalisé et disposé de manière à guider un dispositif d'accouplement correspondant (94) à l'extrémité proximale (92) d'un dispositif de transfert de force (90) au niveau d'une position à l'intérieur de la région de travail.

4. Dispositif de manipulation (20) selon l'une quelconque des revendications précédentes, dans lequel le dispositif de guidage comprend une surface de glissement (32, 34) pour guider un dispositif d'accouplement correspondant (94) à l'extrémité proximale (92) d'un dispositif de transfert de force (90).

5. Dispositif de manipulation (20) selon la revendication précédente, dans lequel la surface de glissement (32, 34) est disposée au niveau d'un bord d'une nervure (31, 33) au niveau du premier composant (30).

6. Dispositif de manipulation (20) selon la revendication précédente, dans lequel le deuxième composant (50) présente une fente (52) dans laquelle est disposée la nervure (31, 33) au niveau du premier composant (30).

7. Dispositif de manipulation (20) selon la revendication précédente, dans lequel le dispositif d'accouplement (54) au niveau du deuxième composant (50) comprend deux rainures (54) parallèles et opposées l'une à l'autre dans deux parois (67) opposées l'une à l'autre de la fente (52).

8. Dispositif de manipulation (20) selon l'une quelconque des revendications 4 à 7, dans lequel la surface de glissement (32, 34) présente la forme d'une pièce découpée dans une surface d'enveloppe d'un cylindre circulaire.

9. Dispositif de manipulation (20) selon la revendication précédente, dans lequel le dispositif d'accouplement (54) est réalisé pour l'accouplement à un dispositif d'accouplement de forme sphérique correspondant (94) à l'extrémité proximale (92) d'un dispositif de transfert de force (90),
la largeur de la nervure (31, 33) et la largeur de la fente (52) sont respectivement inférieures au diamètre du dispositif d'accouplement de forme sphérique correspondant (94) à l'extrémité proximale (92) d'un dispositif de transfert de force (90) pour lequel est prévu le dispositif de manipulation (20).

10. Dispositif de manipulation (20) selon l'une quelconque des revendications précédentes, dans lequel le dispositif de guidage comprend deux surfaces de glissement opposées l'une à l'autre (32, 34) qui sont réalisées et disposées de manière à guider entre elles un dispositif d'accouplement correspondant (94) à l'extrémité proximale (92) d'un dispositif de transfert de force (90).

11. Dispositif de manipulation (20) selon la revendication précédente, dans lequel les surfaces de glissement (32, 34) présentent la forme de deux pièces découpées dans une surface d'enveloppe d'un cylindre circulaire.

12. Instrument médical démontable (10), comprenant :
un dispositif de manipulation (20) selon l'une quelconque des revendications précédentes ;
une tige (80), dont l'extrémité proximale (82) est connectée ou peut être connectée de manière mécaniquement démontable au dispositif de manipulation (20) ;
un dispositif de transfert de force (90) avec un dispositif d'accouplement (94) à l'extrémité proximale (92) du dispositif de transfert de force (90), correspondant au dispositif d'accouplement (54) au niveau du deuxième composant (50).

13. Procédé de fabrication d'un dispositif de manipulation (20) pour former un instrument médical démontable (10) selon la revendication 12, comprenant les étapes suivantes :
réalisation (101) d'un premier alésage (42) dans un premier composant (30), le premier alésage (42) étant prévu et réalisé pour recevoir une région proximale (91) d'un dispositif de transfert de force (90) ;
enlèvement (102) de matière au niveau du premier composant (30) depuis deux côtés opposés l'un à l'autre jusqu'à deux surfaces planes parallèles (47), qui sont parallèles à l'axe (44) du premier alésage (42) et qui coupent la surface intérieure (43) du premier alésage (42) ;
réalisation (103) d'un deuxième alésage (62) dans un deuxième composant (50), le deuxième alésage (62) étant prévu et réalisé pour recevoir un dispositif d'accouplement (94) à l'extrémité proximale (92) d'un dispositif de transfert de force (90) ;
réalisation (104) d'une fente (52) dans le deuxième composant (50), la fente (52) étant parallèle à un axe (64) du deuxième alésage (62) et la largeur de la fente (52) étant inférieure au diamètre du deuxième alésage (62) ;
connexion (105) du premier composant (30) et du deuxième composant (50) de telle sorte que le deuxième composant (50) puisse être déplacé par rapport au premier composant (30).
